(19) Europäisches Patentamt
European Patent Office
Office européen des brevets

(11) Publication number: **0 531 733 A1**

# (12) EUROPEAN PATENT APPLICATION

(21) Application number: **92113744.4**

(51) Int. Cl.$^5$: **C12N 5/00**

(22) Date of filing: **12.08.92**

(30) Priority: **12.08.91 JP 202008/91**

(43) Date of publication of application:
**17.03.93 Bulletin 93/11**

(84) Designated Contracting States:
**AT CH DE FR GB IT LI SE**

(71) Applicant: **SAKAI ENEX KABUSHIKI KAISHA**
**15-1, Hanandonaka 2-chome**
**Fukui-shi, Fukui-ken(JP)**

(72) Inventor: **Ohno, Masano**
**185, Green Heights 10-chome, Shimizu-cho**
**Nyu-gun, Fukui-ken(JP)**
Inventor: **Yasuda, Kimiaki**
**129, Heisei, Matsuoka-cho**
**Yashida-gun, Fukui-ken(JP)**
Inventor: **Matsumura, Masatoshi**
**1250-2, Koya**
**Tsukuba-shi, Ibaraki-ken(JP)**

(74) Representative: **Hansen, Bernd, Dr.**
**Dipl.-Chem. et al**
**Hoffmann, Eitle & Partner Patent- und**
**Rechtsanwälte Arabellastrasse 4 Postfach**
**81 04 20**
**W-8000 München 81 (DE)**

## (54) Carrier for animal cell culture.

(57) A carrier for animal cell culture, comprising a base material having a three-dimensional network structure, wherein a cell adhesive factor and a cell growth factor are immobilized on a surface of the carrier. The carrier for animal cell culture according to the present invention enables the function and growth of cells to be positively promoted after animal cells are adhered to the carrier.

EP 0 531 733 A1

## BACKGROUND OF THE INVENTION

1. Field of the Invention

The present invention relates to a carrier for animal cell culture and a process for producing the same. More particularly, the present invention is concerned with a carrier for animal cell culture wherein a factor for adhering animal cells to the surface of the carrier and a factor for promoting the growth etc. of the adhered cells are immobilized on the surface thereof.

2. Description of the Related Art

When animal cells are cultivated in a large amount for the purpose of producing a physiologically active substance, most of the cells, except for some cell lines capable of growing in suspension cannot grow without adhering to a solid surface. In some cells which are inherently easy to adhere to the surface of a solid, the introduction of a foreign gene thereinto through gene manipulation or the like has often caused the adhesivity to deteriorate.

Under the above, various proposals have been made to develop a suitable carrier for animal cell culture. Efforts have been made to attain the following two objects with respect to a highly efficient animal cell cultivation. The first object is to increase the surface area available for cell adhesion, and second object is to increase the efficiency of cell adhesion to the surface of the carriers.

To achieve a large surface area, methods using solid microcarreirs and three-dimensional porous structure have been utilized and proposed, the former referring to tightly packed structure having no void spaces inside, while the latter to a matrix with open and continuous pores allowing liquid to pass through.

On the other hand, to attain the second object, it is, for example, a common practice to add an animal serum to a culture medium since cell adhesive proteins such as fibronectin and vitronectin are present in animal serum. Further, an attempt has been made to treat the carrier surface for the purpose of electrostatically attracting cells and to coat the carrier with collagen or the like for the purpose of imparting a cell adhesivity through the biological affinity of collagen.

These proposals have enabled or enhanced cell adhesion to the surface of a carrier. However, the cells adhered to the carrier should maintain their function and, in some cases, growth. The term "growth" used in the present specification means the promotion of cell division. After adhesion to surface-modified carriers described above whether or not the cells can maintain the function and growth depends upon the properties of the cells per se. Specifically, problems do not often occur in the case of cells capable of maintaining the function and growth after the adhesion to a carrier, while it is not easy to cultivate cells whose functions and growth are hardly maintained after adhesion. Even in the case of cells capable of maintaining the function and easily growing, the reliance upon properties of the cells per se is often insufficient to obtain a desired product with a high efficiency through a high-density culture.

## SUMMARY OF THE INVENTION

An object of the present invention is to provide a carrier for animal cell culture which can maintain as well as enhance the function of cells and promote cell growth after cells adhered to a carrier.

Another object of the present invention is to provide a carrier for animal cell culture which enables animal cells to be cultivated in a higher density and a desired product to be produced with a higher efficiency.

The present invention provides a carrier for animal cell culture, comprising a base material having a three-dimensional network-type porous structure, wherein a cell adhesive factor and a cell growth factor are immobilized on a surface of the carrier.

## DESCRIPTION OF THE PREFERRED EMBODIMENTS

### Cell Adhesive Factor and Cell Growth Factor

The cell adhesive factor held on the surface of a carrier for animal cell culture according to the present invention is a factor for enabling the cell adhesivity inherent in the cells, that is, a property such that cells adhere to each other or to the surface of a solid other than cells, to be exhibited. Examples of the factor include proteins such as fibronectin, vitronectin, fibrinogen, laminin, collagen, gelatin and fetuin. These proteins specifically bind to a receptor present on the surface of cells considered responsible for the

adhesion of the cells to the surface of the solid. The carrier for animal cell culture which holds the factor on the surface thereof enables animal cells to be effectively adhered to the surface of the carrier.

The immobilization of the cell adhesive factor on the surface of the carrier can be realized, for example, by coating the surface of a base material of the carrier for animal cell culture with the above factor per se or after the dissolution or dispersion of the factor in a suitable liquid or a solid carrier. With respect to coating through the use of collagen as protein, reference may be made to a method described in, for example, Japanese Patent Application No. 242246/1990, the teachings of which are hereby incorporated by reference. Further, it is also preferred to chemically link the factor to the surface of a carrier through the utilization of a functional group present in the molecule of the factor.

According to a further preferred embodiment of the present invention, it is also useful to link only an active site of the cell adhesive factor to the base material of the carrier. For example, the active site of some of the above-described proteins is known as RGD which, more specifically, contains an amino acid sequence represented by Arg-Gly-Asp. Therefore, a peptide containing the RGD sequence may be linked to the surface of the carrier to prepare a carrier for animal cell culture on which a cell adhesive factor is held. The expression "peptide containing the RGD sequence" used herein is intended to mean that a peptide comprising one or more amino acid residues may further be contained on the N and/or C terminal sides of the RGD sequence as far as the activity as a cell adhesive factor is not spoiled. The linkage to the carrier may be conducted through a functional group present in the molecule of the peptide containing RGD.

The expression "cell growth factor held on the surface of a carrier for animal cell cultivation" used in the present invention is intended to mean a factor which acts on cells to regulate the growth and, in some cases, differentiation of cells. Specific examples of such a cell growth factor include hexamethasone, insulin, glucagon, transferrin, asialoglycoprotein, epidermal growth factor (EGF), platelet derived growth factor (PDGF), fibroblast growth factor family (FGF-family), insulin-like growth factor (IGF), endothelial growth factor (ECGF) and heparin. The abovementioned substances are specifically bound to receptors present on the surface of cells. The carrier for animal cell culture whose surface are modified with the said growth factors according to this invention, are thus able to promote positive growth and cause differentiation of the animal cells adhered to the carrier.

As in the cell adhesive factor, the cell growth factor can be immobilized on the surface of the carrier by directly coating the factor on the surface of the carrier. It is also preferred to chemically link the factor to the surface of the carrier through the utilization of a functional group present in the molecule of the factor.

According to a further preferred embodiment of the present invention, in the case of the said cell growth factors as well, it is useful to link only the active site to the base material of the carrier. For example, an amino acid sequence -Arg-Gly-Phe-Phe- linked to the surface of the base material can serve as a ligand for an insulin receptor present on the surface of animal cells. The base material whose surface is linked with the said amino acid sequence can then serve as an animal cell carrier having a cell growth factor. Other specific examples of the active site of the cell growth factor include a galactose side chain of asialoglycoprotein.

The term "cell growth factor" used in the present invention is intended to mean not only a factor for regulating the growth of cells but also a factor for allowing the cells to exhibit a particular function.

As described above, the cell adhesive factor and the cell growth factor may be directly held or linked to the surface of the carrier. It is still preferred for them to be linked to the surface of the carrier through the so-called "spacer" which enables the base material and the factors to be held together while keeping them apart at a given distance and maintaining a three-dimensional degree of freedom. In the carrier for animal cell culture according to the present invention, when the cell adhesive factor and the cell growth factor serve as a ligand for a receptor present on the surface of cells, the linkage between these factors and the receptors is considered indispensable to the achievement of the functions. However, the receptor for these factors is not always present near the surface of cells which is in contact with the carrier. In this connection, it is advantageous for factors to be held on the surface of the carrier through a spacer because the factors can bind to the receptor even when the receptor is absent in the vicinity of the surface of cells in contact with the carrier. Although the distance between these factors and the carrier, that is, the length of the spacer, can be properly determined by taking the kinds of factors and cells and the like into consideration, for example, the length is preferably about 2 nm. The expression "holding the factors while maintaining a three-dimensional degree of freedom" is intended to mean a degree of freedom such that the factors can freely change the position within a given space without being fixed to a particular position.

According to a further preferred embodiment of the present invention, a polymer compound is suitable as the spacer. Preferred examples of the polymer compound include polyethyleneimine, polyamino acid and polymethylene. Among these polymeric materials, those having a group which can be converted to a

cationic group upon being brought into contact with a culture solution (that is, water) is preferred. The presence of a cationic group is advantageous because cells are first adsorbed to the surface through electrostatic force. From this point of view, the use of polyethyleneimine as the spacer is preferred in the present invention. According to a further preferred embodiment, it is advantageous to use a highly branched polyethyleneimine. When the polymer compound as a spacer is highly branched, the factors can be advantageously linked to a plurality of side chains. Further, in this case, when the factors are linked to the carrier through a plurality of side chains in the same molecule, the polymer compound does not advantageously liberate from the carrier with ease. A polyethyleneimine comprising a unit structure represented by the following formula (I) is particularly preferred.

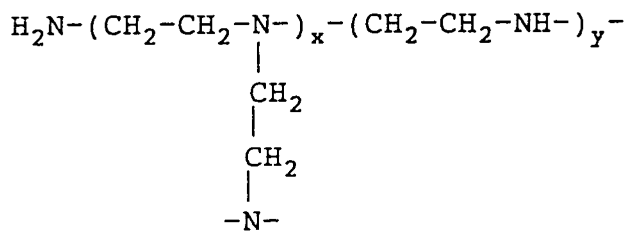

(I)

wherein X and Y are each a natural number of 1 or more.

Although the molecular weight of the polyethyleneimine may be properly determined by taking the length of the spacer into consideration, it is 3,000 or more from the practical point of view, preferably about 3,000 to 100,000.

The linkage of the spacer with the cell adhesive factor and the cell growth factor and the carrier can be properly practiced through the utilization of a functional group present in the factors, space and carrier.

Base Material for Carrier

The carrier for animal cell culture according to the present invention has a three-dimensional network structure. The term "three-dimensional network structure" used herein is intended to mean a structure having continuous pores having such a size that animal cells can be cultivated therein.

According to a further preferred embodiment of the present invention, the base material comprises a foam having a porosity of 80% or more, preferably 95% or more, still preferably 97% or more and a mean pore diameter of 0.03 to 2.0 mm, preferably 0.1 to 1.5 mm. When the porosity and mean pore diameter of the base material are in the above-described respective ranges, cells grow, and no necrosis occurs even in cells present in the deep part of the carrier. On the other hand, when the mean pore diameter is about 0.03 mm or less, it becomes substantially impossible for many animal cells to be cultivated within the carrier.

The carrier for animal cell culture according to the present invention holds the cell adhesive factor and the cell growth factor on its surface. The thickness of a layer newly formed by immobilizing these factors is negligible, and the porosity and mean pore diameter of the base may be regarded as the porosity and mean pore diameter of the carrier.

The base material preferably comprises a foam of a naturally occurring polymer, for example, cellulose. The cellulose may be any of natural cellulose (crystal form: type I) and regenerated cellulose (crystal form: type II). When the base material is highly elastic as in a foam, a further reduction in the effect of carrier collision on the cells can be advantageously attained.

The cellulose foam which is preferred as the base material is prepared, for example, by subjecting a high-purity pulp produced from wood to a chemical treatment to give a viscose, adding and mixing a third component to form pores with the viscose, pouring the mixture into a mold, heating and solidifying the mixture and suitably removing the third component. A substance which can be easily removed after the formation of a foam is used as the third component. Examples of the third component include crystalline materials, waxes, surfactants having a low HLB value and sublimable substances which are insoluble or sparingly soluble in an aqueous solution. The third component can be conducted by a method properly selected from extraction with an organic solvent, sublimation by heating and other methods depending upon properties of the third component. According to this method, a base material having desired properties (porosity, pore diameter, etc.) can be easily prepared through proper selection of the size, kind and amount of blending of the third component.

The porosity and mean pore diameter of the base material can be measured by a pressure injection method and a BET method, for example, methods described in "Tako Zairyo Handobukku (Porous Material

Handbook)" prepared under the supervision of Jun Kamisawa and Masanobu Kaya and published by IPC (1988), the teachings of which are hereby incorporated by reference.

According to a particularly preferred embodiment of the present invention, the base material comprises a cellulose foam and has a porosity of 97% or more, a mean pore diameter of 0.5 to 1.5 mm and a specific gravity of 1.4 to 1.7 g/cm$^3$. Example of the base material having such properties include one commercially available under the trade name of SIC-CS from Sakai Engineering Co., Ltd., Fukui, Japan.

The carrier for animal cell culture according to the present invention is used after molding into a molder having a shape and a size in conformity with various culture methods. The mean particle diameter of the carrier is preferably about 1 to 5 mm, and the carrier is preferably in the form of a cube, a rectangular parallelepiped and a globular shape. In the carrier for animal cell culture according to the present invention, when the base material comprises a foam, it is a common practice for the foam to be chopped into small pieces. Although the cell adhesive factor and the cell growth factor may be immobilized before or after the base material is chopped into a desired size, it would be preferable for the factors to be immobilized after chopping from the viewpoint of easiness of the procedure.

Culture of Animal Cells

The carrier for animal cell culture according to the present invention has a surface which allows an excellent adhesion and promotes growth of unlimited type of cells and can be applied also to serum-free cultivation. Specific examples of the animal cells which may be adhered and growth in these carriers include fibroblasts such as human fetal preputial fibroblasts, lung fibroblasts of Chinese hamsters, avian fetal fibroblasts and neonatal kidney cells of Syrian hamsters, epithelial cells such as carcinoma cells of human uterine cervix, ovarian cells of Chinese hamsters, breast carcinoma cells of mice and kidney cells of African green monkeys and vascular endothelial cells. The particular advantage of the carrier for animal cell culture according to the present invention is its application to the culture of cells which poorly adhere to a carrier and whose functions are either lost or altered after adhesion to a carrier, for example, primary cells such as canine hepatocytes, human coronary epithelial cells, golden hamster Langerhans' cells, rat thymic epithelial cells and rat kidney epithelial cells, and some transformed cells having lowered adhesivity.

The carrier for animal cell culture according to the present invention can be utilized in the same manner as that of the conventional carrier and enables cells to be cultivated in a higher density and a larger quantity as compared with the conventional carrier.

The present invention will now be described in more details with reference to the following examples, though it is not limited to these examples only.

Example 1

1) Cell Adhesive Factor, Cell Growth Factor and Spacer

RGD was prepared as a cell adhesive factor as follows. A peptide of a sequence represented by Gly-Arg-Gly-Asp-Ser-Gly was synthesized by a solid support synthesis method. The synthesized peptide was subjected to crude purification by gel filtration according to the conventional method and then purified by HPLC.

Insulin (a product of Sigma Chemical Co.; about 24 I.U./mg; Zn content: about 0.5%) was used as a cell growth factor.

A polystyreneimide (Epomin SP-200 manufactured by Nippon Shokubai Kagaku Kogyo Co., Ltd.) was used as a spacer. The molecular weight of the polymer was measured by a limiting viscosity method and found to be 10,000.

2) Preparation of Base Material for Carrier

A cellulose foam (manufactured by Sakai Engineering Co., Ltd.) having a porosity of 97%, a mean pore diameter of 1.05 mm and a true specific gravity of 1.52 g/cm$^3$ was used as a base material for a carrier having a three-dimensional network structure. The foam was used after chopping into a size of about 1.0 mm$^2$.

3) Preparation of Carrier for Cultivation of Animal Cells

At the outset, the polyethyleneimine was linked to the base material comprising a cellulose foam as follows. 1.0 g of a macerated cellulose foam was crosslinked with epichlorohydrin, and the foam was reacted with 1.2 g of polyethyleneimine in a DMF solvent for 24 hr. With respect to the amount of a primary amine linked to the carrier, the amount of nitrogen was measured by a semi-micro Kjeldahl method, and the estimated amount of nitrogen per unit weight of the carrier was calculated.

Subsequently, the cell adhesive factor and the cell growth factor were linked to polyethyleneimine linked to the base material. The procedure of the reaction was as follows.

1 g of the carrier was mixed with 0.1 mmol of the cell adhesive factor in DMSO, and 1.1 equivalents, based on the cell adhesive carrier, of 1-ethyl-3-(3-dimethylaminopropyl)carbodiimide hydrochloride as a condensing reagent was added thereto. The mixture was shaken for 24 hr. The reaction mixture was sufficiently washed with water. 10 mg of the cell growth factor was dissolved in a buffer solution having a pH value of 3 and added to 1 g of the carrier, and 10 equivalents, based on the cell growth factor, of 1-ethyl-3-(3-dimethylaminopropyl)carbodiimide hydrochloride as a condensing reagent was added thereto. The mixture was further shaken for 24 hr. The carrier was sufficiently washed with water, and the amount of linkage of each factor was quantitatively determined by a residual method. Specifically, the cell adhesive factor in the residual solution and the cell growth factor in the residual solution were quantitatively determined by an amino acid analysis method and RIA (isotope labelling immunological quantitative determination method), respectively, and calculating the difference between the initial amount and the amount after the reaction of each factor. As a result, the amounts of linkage of the cell adhesive factor and the cell growth factor were 0.2 mmol/g-carrier and 6 mg/g-carrier, respectively.

Examples 2 to 5

Carriers for animal cell culture were produced in the same manner as that of Example 1, except that the following cell adhesive factors and cell growth factors were used.

| Ex. | Cell adhesive factor | Cell growth factor |
|---|---|---|
| 2 | Arg-Gly-Asp-Ser-Gly | Arg-Gly-Phe-Phe |
| 3 | Arg-Gly-Asp-Ser-Gly | glucagon |
| 4 | Arg-Gly-Asp-Ser-Gly | glucagon-like peptide |
| 5 | Arg-Gly-Asp-Ser-Gly | epithelial cell growth factor (EGF) |

As with Arg-Gly-Asp-Ser-Gly in Example 1, Arg-Gly-Phe-Phe was synthesized by a solid support synthesis method. Glucagon was an extract (manufactured by Sigma Chemical Co.) from a mixture of a bull pancreas with a pig pancreas. Further, use was made of a glucagon-like peptide comprising a sequence of His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Ala-Lys-Glu-Phe-Ile-Ala-Trp-Leu-Val-Lys-Gly-Arg-Gly (manufactured by Sigma Chemical Co.). mEGF (manufactured by Collaborative Research) produced from mouse submandibular gland was used as the epithelial cell growth factor.

Comparative Examples 1 to 3

Carriers for animal cell culture were produced in the same manner as that of Example 1, except that no cell growth factor was linked to the base material (Comparative Example 1), no cell adhesive factor was linked to the base material (Comparative Example 2) and neither cell growth factor nor cell adhesive factor was linked to the base material (Comparative Example 3).

Culture of Cells

Cells derived from recombinant Chinese hamster ovarium (r-CHO) having a weakened cell adhesivity and canine hepatocytes were cultivated through the use of carriers for animal cell culture prepared in Examples 1 to 5 and Comparative Examples 1 to 3. A spinner flask (250 ml; a product of Bellco) equipped with a paddle blade agitator was used as a culture vessel. The medium used was an SF-02 medium (manufactured by Sanko Pharmaceuticals, Inc.) in the case of r-CHO and a William E medium containing 10% of a fetal calf serum in the case of the hepatocytes.

1.2 g of a sterilized, dried carrier was placed in a flask and swollen in 50 ml of PBS ($Ca^{2+}$(-), $Mg^{2+}$(-)) for 3 hr. Thereafter, the carrier was washed three times with the above-described medium to be used in the culture. After washing, the medium was discarded by decantation, and inoculation of cells was conducted. 4 $\times$ $10^7$ cells were well suspended in 40 ml of the medium. the cell suspension was scattered on the carrier by means of a pipette so that the suspension was sucked in the carrier. The system was allowed to stand in a 5% $CO_2$ incubator at 37°C for 3 hr while conducting intermittent agitation every 30 min, thereby conducting adhesion of cells. Then, the remaining medium (160 ml) was added to make the total volume 200 ml, and the cultivation was initiated in a 5% $CO_2$ incubator at 37°C. The density of inoculation of cells was 2 $\times$ $10^5$ cells based on the final culture solution.

The degree of adhesion of cells and the degree of growth were evaluated as follows.

Evaluation of Cell Adhesion:

In the above procedure, the medium was allowed to stand in a $CO_2$ incubator for 3 hr (before the remaining medium (160 ml) was added), and 0.5 ml of the medium was sampled and subjected to counting by means of a hematocytometer. The percentage cell adhesion was calculated by the following equation.

$$\text{Cell adhesion (\%)} = \frac{4 \times 10^7 \text{ (cells)} - \text{cell density of sample (cells/ml)} \times 40}{4 \times 10^7 \text{ (cells)}} \times 100$$

Evaluation of Growth of Cells:

Four days after the initiation of culture, several adhered carriers were sampled, and counting of the number of cells was conducted. The carrier was sufficiently washed with PBS ($Ca^{2+}$(-), $Mg^{2+}$(-)), immersed in a solution of trypsin dissolved in PBS ($Ca^{2+}$(-), $Mg^{2+}$(-)) and incubated at 37°C for 30 min while intermittently agitating the system. Thereafter, the carrier was disintegrated by means of a spatula to discharge the cells outside the carrier. The resultant cell suspension was extracted by means of a pipette and suspended in an equal amount of the medium. The suspension was centrifuged, and the supernatant was discarded. The survival rate of the cells adhered to the carrier was measured by staining with trypan blue. Since 1 g of the dried carrier corresponds to 15,000 carriers having a size of 1.0 mm square and the number of carriers per 1 ml of the culture solution is 15,000 carriers $\times$ 1.2 g/200 = 90 carriers/ml, the density of the culture standard solution was determined by the following equation.

$$\text{Cell density (cells/ml)} = \frac{\text{90 carriers/ml}}{\text{number of sampled carriers}} \times \text{total number of cells peeled after treatment with trypsin}$$

Table 1

| Cells derived from recombinant Chinese hamster ovarium (r-CHO) | | | |
|---|---|---|---|
| Carrier | Cell adhesion (%) | Cell density after 4 days (cells/ml) | Survival rate after 4 days (%) |
| Ex. 1 | 97.8 | $6.8 \times 10^6$ | 89.3 |
| Ex. 2 | 98.7 | $5.7 \times 10^6$ | 91.5 |
| Ex. 3 | 98.0 | $7.2 \times 10^6$ | 89.4 |
| Ex. 4 | 96.9 | $5.9 \times 10^6$ | 90.6 |
| Ex. 5 | 97.4 | $6.2 \times 10^6$ | 89.4 |
| Comp.Ex. 1 | 98.5 | $1.8 \times 10^6$ | 91.2 |
| Comp.Ex. 2 | 92.3 | $2.9 \times 10^6$ | 90.6 |
| Comp.Ex. 3 | 91.6 | $1.1 \times 10^6$ | 91.3 |

Table 2

| Canine hepatocytes | | | |
|---|---|---|---|
| Carrier | Cell adhesion (%) | Cell density after 4 days (cells/ml) | Survival rate after 4 days (%) |
| Ex. 1 | 96.3 | $4.0 \times 10^6$ | 90.5 |
| Ex. 2 | 98.8 | $2.7 \times 10^6$ | 92.1 |
| Ex. 3 | 97.2 | $4.7 \times 10^6$ | 91.1 |
| Ex. 4 | 97.0 | $2.2 \times 10^6$ | 90.6 |
| Ex. 5 | 96.7 | $3.6 \times 10^6$ | 88.9 |
| Comp.Ex. 1 | 97.7 | $2.0 \times 10^6$ | 92.1 |
| Comp.Ex. 2 | 91.1 | $7.4 \times 10^6$ | 92.4 |
| Comp.Ex. 3 | 90.5 | $1.2 \times 10^6$ | 91.8 |

## Claims

1. A carrier for animal cell culture, comprising a base material having a three-dimensional network structure, wherein a cell adhesive factor and a cell growth factor are immobilized on a surface of said carrier.

2. A carrier for animal cell culture according to claim 1, wherein said cell adhesive factor is fibronectin, vitronectin, fibrinogen, gelatin, laminin or collagen.

3. A carrier for animal cell culture according to claim 1, wherein said cell adhesive factor is a peptide containing an amino acid sequence of Arg-Gly-Asp.

4. A carrier for animal cell culture according to claim 1, wherein said cell growth factor is hexamethasone, insulin, glucagon, EGF, PDGF, IGF, FGF-family or heparin.

5. A carrier for animal cell culture according to claim 1, wherein said cell growth factor is a peptide containing an amino acid sequence of Arg-Gly-Phe-Phe.

6. A carrier for animal cell culture according to any one of claims 1 to 5, wherein said cell adhesive factor and said cell growth factor are linked to said base material through a spacer.

7. A carrier for animal cell culture according to claim 6, wherein said spacer is a polymer.

8. A carrier for animal cell culture according to claim 7, wherein said spacer is a polyethyleneimine having a molecular weight of 3,000 or more.

**9.** A carrier for animal cell culture according to claim 1, wherein said base material comprises a foam having a porosity of 80% or more and a mean pore diameter of 0.05 to 2.0 mm.

**10.** A carrier for animal cell culture according to claim 9, wherein said base material comprises a naturally occurring polymer having a particle diameter of 1.0 to 5.0 mm, a porosity of 97% or more, a mean pore diameter of 0.3 to 2.0 mm and a specific gravity of 1.4 to 1.7 g/$cm^3$.

European Patent Office

# EUROPEAN SEARCH REPORT

Application Number

EP 92 11 3744
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X<br><br>Y | WO-A-9 101 760 (UNITED STATES GOVERNMENT)<br>* the whole document * | 1-2,4<br><br>6-8 | C12N5/00 |
| Y | WO-A-8 905 616 (BIO-METRIC SYSTEMS, INC.)<br>* the whole document * | 6-8 | |
| Y | WO-A-9 105 036 (BOARD OF REGENTS, THE UNIVERSITY OF TEXAS SYSTEM)<br>* claims * | 1-4 | |
| Y | PATENT ABSTRACTS OF JAPAN<br>vol. 15, no. 52 (C-803)(4580) 7 February 1991<br>& JP-A-22 83 277 ( UBE NITTO KASEI CO. LTD. ) 20 November 1990<br>* abstract * | 1-4 | |
| Y | EP-A-0 416 586 (MILLIPORE CORPORATION)<br>* the whole document * | 1-4,9-10 | |
| | | | TECHNICAL FIELDS SEARCHED (Int. Cl.5) |
| Y | EP-A-0 420 171 (KIRIN BEER KABUSHIKI KAISHA)<br>* claims * | 1-4,9-10 | C12N<br>C07K |
| P,X<br><br>P,Y | PATENT ABSTRACTS OF JAPAN<br>vol. 16, no. 351 (C-968)(5394) 29 July 1992<br>& JP-A-41 08 377 ( UBE NITTO KASEI CO. LTD. ) 9 April 1992<br>* abstract * | 1-2,4<br><br>3,6-10 | |

-/--

The present search report has been drawn up for all claims

| Place of search | Date of completion of the search | Examiner |
|---|---|---|
| THE HAGUE | 07 JANUARY 1993 | BEVAN S.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 11 3744
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,Y | DATABASE WPIL<br>Section Ch, Week 9231,<br>Derwent Publications Ltd., London, GB;<br>Class A96, AN 92-255414<br>& JP-A-4 173 086 (KIRIN BREWERY KK. & SAKAI ENG. KK.) 19 June 1992<br>* abstract *<br>--- | 3,6-10 | |
| A | EP-A-0 171 887 (ELI LILLY AND COMPANY)<br>* abstract *<br>--- | 4-5 | |
| A | US-A-4 332 916 (B.P. THILL)<br>* the whole document *<br>----- | 7-10 | |

TECHNICAL FIELDS SEARCHED (Int. Cl.5 )

The present search report has been drawn up for all claims

| Place of search | Date of completion of the search | Examiner |
|---|---|---|
| THE HAGUE | 07 JANUARY 1993 | BEVAN S.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document